# EUROPÄISCHE PATENTSCHRIFT

(11) **EP 1 586 656 B1**
(45) Veröffentlichungstag und Bekanntmachung des Hinweises auf die Patenterteilung: **28.11.2007**
(21) Anmeldenummer: 05005591.2
(22) Anmeldetag: 15.03.2005
(51) Int. Cl.: C12Q 1/24, G01N 33/50

(54) **Verfahren zur Bestimmung der Photosynthese-Aktivität von Algen**
Determination of the photosynthesis activity of algae
Détermination de l'activité photosynthétique d'algues

(30) Priorität: 15.03.2004 DE 102004012484
(43) Veröffentlichungstag der Anmeldung: 19.10.2005
(73) Patentinhaber: INNO-Concept GmbH, 15344 Strausberg (DE)
(72) Erfinder: Karasyova, Tatyana, 12683 Berlin (DE); Smolkova, Olga, 700000 Taschkent (UZ); Lutsenko, Oksana, 10369 Berlin (DE)
(74) Vertreter: Kruspig, Volkmar

(56) Entgegenhaltungen:
- DE-A1- 4 332 290
- NAESSENS M ET AL: "Whole-cell biosensor for direct determination of solvent vapours" BIOSENSORS AND BIOELECTRONICS 01 MAR 1998 UNITED KINGDOM, Bd. 13, Nr. 3-4, 1. März 1998 (1998-03-01), Seiten 341-346, XP002337652 ISSN: 0956-5663
- GANDER SARAH ET AL: "The development of a small-scale biofilm model suitable for studying the effects of antibiotics on biofilms of Gram-negative bacteria" JOURNAL OF ANTIMICROBIAL CHEMOTHERAPY, Bd. 40, Nr. 3, September 1997 (1997-09), Seiten 329-334, XP002337654 ISSN: 0305-7453
- FRENSE D ET AL: "DETECTION OF ENVIRONMENT POLLUTANTS USING OPTICAL BIOSENSOR WITH IMMOBILIZED ALGAE CELLS" SENSORS AND ACTUATORS B, ELSEVIER SEQUOIA S.A., LAUSANNE, CH, Bd. B51, Nr. 1/2/3, 31. August 1998 (1998-08-31), Seiten 256-260, XP000669834 ISSN: 0925-4005
- MERZ DANIELA ET AL: "Chlorophyll fluorescence biosensor for the detection of herbicides" FRESENIUS' JOURNAL OF ANALYTICAL CHEMISTRY, Bd. 354, Nr. 3, 1996, Seiten 299-305, XP002206792 ISSN: 0937-0633

## Beschreibung

Die Erfindung betrifft ein Verfahren zur Bestimmung der Vitalität von Algen durch Sauerstoff-Freisetzung bei der Photosynthese durch Messung deren Sauerstoff-Bilanz im künstlich angeregten Photosyntheseprozess einer definierten Algenprobe gemäß Oberbegriff des Patentanspruchs 1.

Zur Bestimmung der Vitalität von Pflanzen ist es bekannt, deren Sauerstoff-Freisetzung unter definierten Bedingungen zu untersuchen. Beispielsweise wird hierfür ein Teil des zu untersuchenden Blattes in einen speziellen Probenraum einer Untersuchungseinrichtung verbracht, um dort unter definierter Bestrahlung bei vorgegebener Temperatur einer CO₂-gesättigten Atmosphäre ausgesetzt zu werden. Über eine spezielle Sonde wird dann die sich ergebende O₂-Emission gemessen. Eine derartige Photosyntheseprozess-Messeinrichtung nebst zugehörigem Verfahren ist in der DE 43 32 290 A1 offenbart.

Die Photosynthese stellt einen komplexen Prozess dar, mit dessen Hilfe chlorophyllhaltige pflanzliche Organismen organische Substanz und Sauerstoff aus Kohlendioxid und Wasser unter Lichteinwirkung bilden.
Dieser Prozess hängt vom Vegetationstyp und seinem Genotyp sowie vom Einfluss vieler Umweltfaktoren ab. Die Bildung von Sauerstoff ist, wie vorstehend erläutert, direkt messbar. Die Größe der Photosynthese kann entweder nach der Menge des absorbierten Kohlenstoffdioxids oder nach der Menge des freigesetzten Sauerstoffs bestimmt werden.

Während die Bestimmung der Vitalität von Blattpflanzen auf keine nennenswerten Schwierigkeiten bei exakter Behandlung des lebenden Blattmaterials stößt, gestaltet sich die Bestimmung der Sauerstoff-Freisetzung von Algen eher problematisch. Der Stand der Technik in Sachen Probenaufbereitung von Algen ist durch ein Zentrifugieren gekennzeichnet, wobei jedoch durch den Zentrifugationsprozess ein Großteil der Algen abstirbt, so dass eine nachfolgende Vitalitätsbestimmung durch Sauerstoff-Freisetzung keine exakten Ergebnisse liefert.

Ein gattungsgemäßes Verfahren ist aus Naessens, M. et al.; Biosensors & Bioelectronics, Vol. 13 (3-4), S. 341-346 (1998) bekannt. Ein ähnliches Verfahren lehrt Frense, D. et al.; Sensors and Actuators, Vol. 51, S. 256-260 (1998).

Aus dem Vorgenannten ist es daher Aufgabe der Erfindung, ein weiterentwickeltes Verfahren zur Bestimmung der Vitalität von Algen durch Messung deren Sauerstoff-Freisetzung im künstlich angeregten Photosyntheseprozess einer definierten Algenprobe anzugeben, wobei die Probenahme und damit das eigentliche Verfahren zu reproduzierbaren Ergebnissen hoher Signifikanz führen soll.

Die Lösung der Aufgabe der Erfindung erfolgt durch ein Verfahren in seiner Definition gemäß der Lehre nach Patentanspruch 1, wobei die Unteransprüche zweckmäßige Ausgestaltungen und Weiterbildungen darstellen.

Der Grundgedanke der Erfindung liegt demgemäß darin, dass zunächst aus einer Algenlösung mittels Küvette sowie unter Zuhilfenahme einer Saugvorrichtung eine vorgegebene Lösungsmenge entnommen und über ein angefeuchtetes Flächenfilter gezogen wird.

Die in der Lösung befindlichen Algen gelangen somit auf das angefeuchtete Flächenfilter.
Im Anschluss kann ergänzend zum Zweck des sicheren Anhaftens der Algen auf der Filteroberfläche ein Luftstrom über oder durch das Filter geführt werden. Die auf dem Filter anhaftende Algenschicht wird dann zusammen mit dem z.B. blattförmigen Filter in die an sich bekannte Messvorrichtung zur Bestimmung der Sauerstoff-Freisetzung verbracht.

Wie erwähnt, kann das Flächenfilter ein Papier- oder feinporiges Folienmaterial enthalten oder aus einem solchen Material bestehen.

In bevorzugter Ausgestaltung der Erfindung wird als Saugvorrichtung eine handelsübliche Spritze verwendet.

Diese Spritze weist eine zweiteilige Kanüle auf, welche ein austauschbares Filterblatt aufnimmt. Zu diesem Zweck ist der Bereich der Kanüle aufschraubbar zur Aufnahme bzw. Entnahme des Filterblatts ausgeführt.

Zweckmäßigerweise ist dafür Sorge zu tragen, dass sowohl die Algenlösung als auch der alternativ oder ergänzend wirksame Luftstrom so temperiert ist, dass die natürlichen Lebensbedingungen der Algen erhalten bleiben bzw. nicht nachteilig beeinflusst werden.

Die Temperatur der Lösung und der Umgebung liegt im Bereich von im wesentlichen 20°C bis 28°C, vorzugsweise bei 25°C.

Erfindungsgemäß weist die Algenlösung im Wesentlichen 0,25 bis 5 Mio. Algen je ml Lösung auf, wobei die vorgegebene Lösungsmenge der Probe im Bereich zwischen 3 ml bis 8 ml, vorzugsweise bei 5 ml liegt.

Grundsätzlich ist mit der vorbeschriebenen Verfahrensweise sichergestellt, dass sich auf der Filteroberfläche eine Algen-Monoschicht einstellt. Auf diese Weise wird vermieden, dass bei einer mehrschichtigen oder nicht gleichmäßigen, inhomogenen Schichtstruktur sich die Sauerstoff freisetzenden Aktivitäten der Algen behindern oder beeinflussen. Eine Verfälschung der Messergebnisse und der hieraus resultierenden Aussagen über die Vitalität der Algen kann auf diesem Wege weitestgehend ausgeschlossen werden.

Die Erfindung soll nachstehend anhand eines Ausführungsbeispiels sowie von tabellarischen und grafischen Zusammenstellungen typischer Messergebnisse näher erläutert werden.

Hierbei zeigen:
- Tabelle 1: eine Darstellung von Messergebnissen, aus welcher sich die gewünschte Reproduzierbarkeit der Untersuchungen der Algenaktivität ergibt;
- Tabelle 2: eine Darstellung der Abhängigkeit der Änderung des Sauerstoffpartialdrucks von der Algenanzahl in der Lösung nebst grafisch umgesetztem Kurvenverlauf aus dieser Tabelle sowie
- Tabelle 3: eine Aufstellung der Abhängigkeit der Änderung des Sauerstoffpartialdrucks von der Algenzahldichte pro cm³ nebst hieraus abgeleitetem Kurvenverlauf.

Gemäß Ausführungsbeispiel erfolgt die Probenahme der Algen im Vorfeld der eigentlichen Messung wie nachstehend dargelegt.

Zunächst wird dafür gesorgt, dass die Algenlösung unter natürlichen Lebensbedingungen gehalten ist. Für die Entnahme der Algenprobe wird eine Küvette mit Saugvorrichtung und einer geeigneten Filterbefestigungseinrichtung eingesetzt, wobei die Filterbefestigungseinrichtung ein z.B. angefeuchtetes Papier-Filterblatt aufnimmt.

Für die exakte Bestimmung der Algenvitalität mit der erläuterten Vorrichtung wird vorzugsweise eine Menge von 5 ml Algenlösung verwendet. Auf Tabelle 1 sei hierzu verwiesen.

Diese Menge an Lösung wird durch den Filter gesaugt, wobei die in der Lösung vorhandenen Algen auf dem Filter bereits leicht anhaften.

Nach diesem Saugvorgang können die Algen unter Beachtung ihrer Lebensbedingungen durch einen zusätzlichen Luftstrom auf dem Filter zum noch sichereren Haften gebracht werden.
Danach wird der Filter aus der Küvette entnommen und in die Messeinrichtung z.B. eines Photosynthese-Messgeräts vom Typ "PlantVital" (eingetragene Marke) mit der Filterseite zur Beleuchtungseinrichtung für die Sauerstoffbestimmung eingelegt.

Die Bestimmung der Algenanzahl im untersuchten Volumen bzw. die Algenzahldichte erfolgt in den für Algenuntersuchungen üblichen Auszählprozeduren unter Zuhilfenahme eines Mikroskops.
Die Untersuchung der Algenvitalität soll bevorzugt bei einer annähernd gleichbleibenden Temperatur durchgeführt werden, wobei sich hier eine Temperatur von im wesentlichen 25°C als vorteilhaft erwiesen hat.

Die Reproduzierbarkeit der Messungen ist bei exakter Versuchsdurchführung besser als 100±10% (siehe Tabelle 1).

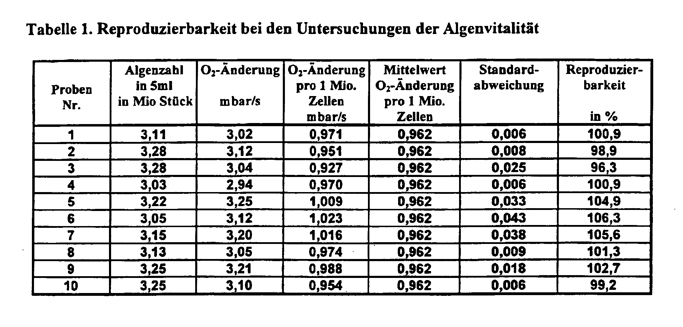

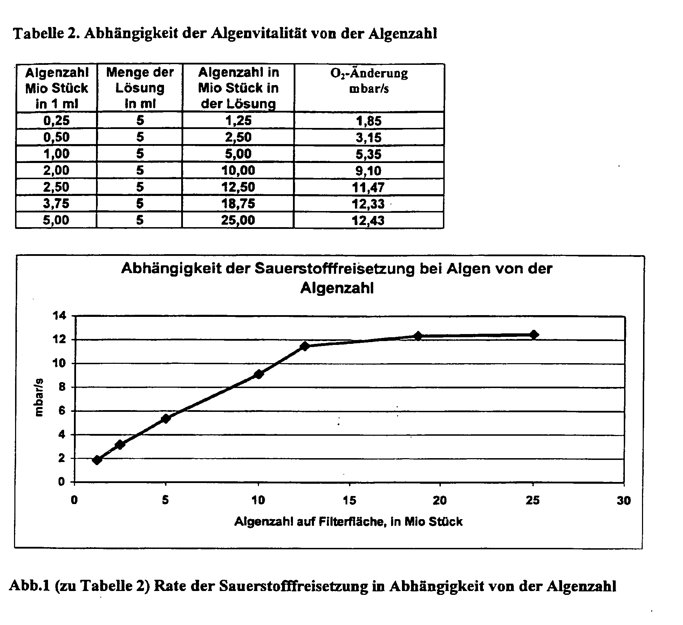

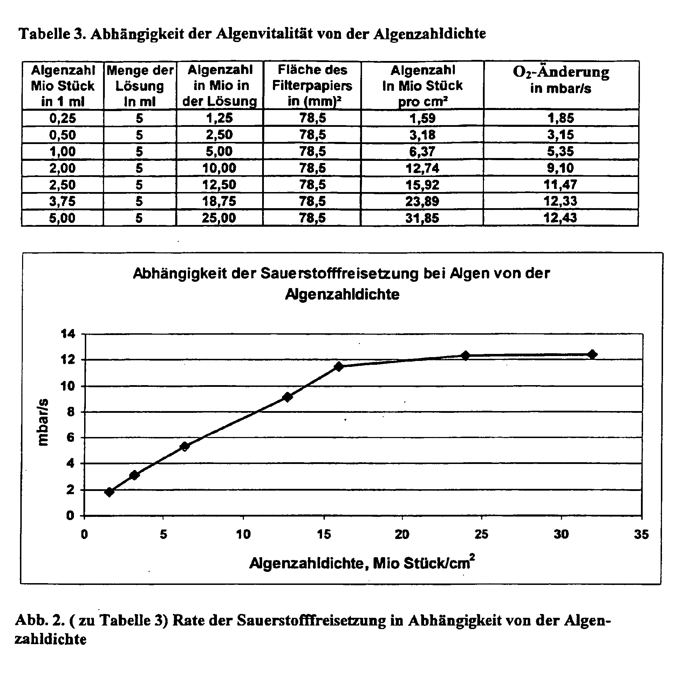

## Patentansprüche

1. Verfahren zur Bestimmung der Vitalität von Algen durch Sauerstoff-Freisetzung bei der Photosynthese-Aktivität durch Messung deren Sauerstoff-Bilanz im künstlich angeregten Photosyntheseprozess einer definierten Algenprobe, wobei aus einer Algenlösung mittels einer Küvette sowie Saugvorrichtung eine vorgegebene Lösungsmenge über ein angefeuchtetes Flächenfilter gezogen wird, so dass die in der Lösung befindlichen Algen auf das Flächenfilter gelangen und hiernach das Filter mit der anhaftenden Algenschicht in die Messeinrichtung zur Bestimmung der Sauerstoff-Bilanz verbracht wird,
**dadurch gekennzeichnet, dass**
die Algenlösung im wesentlichen 0,25 bis 5 Mio. Algen je ml Lösung aufweist, wobei die vorgegebene Lösungsmenge der Probe im Bereich zwischen 3 ml bis 8 ml, vorzugsweise bei 5 ml liegt um auf der Filteroberfläche eine Algen-Monoschicht zu erzeugen.

2. Verfahren nach Anspruch 1,
**dadurch gekennzeichnet, dass**
als Flächenfilter ein angefeuchtetes bzw. feuchtes Papier- oder feinperforiertes Folienmaterial eingesetzt wird.

3. Verfahren nach Anspruch 1 oder 2,
**dadurch gekennzeichnet, dass**
als Saugvorrichtung eine Spritze verwendet wird.

4. Verfahren nach Anspruch 3,
**dadurch gekennzeichnet, dass**
die Spritze eine zweiteilige Kanüle aufweist, welche ein austauschbares Filterblatt aufnimmt.

5. Verfahren nach einem der vorangegangenen Ansprüche,
**dadurch gekennzeichnet,dass**
zum Zweck des verbesserten Anhaftens der Algen auf der Filteroberfläche ein Luftstrom über oder durch das Filter geführt wird.

6. Verfahren nach einem der vorangegangenen Ansprüche,
**dadurch gekennzeichnet, dass**
sowohl die Algenlösung als auch der gegebenenfalls eingesetzte Luftstrom temperiert wird.

7. Verfahren nach Anspruch 6,
**dadurch gekennzeichnet,dass**
die Temperatur der Lösung oder des Luftstroms im Bereich von 20°C bis 28°C, vorzugsweise bei 25°C liegt.

## Claims

1. Method for the determination of the vitality of algae by the release of oxygen during the photosynthesis activity by measuring the oxygen balance thereof in the artificially activated photosynthesis process of a defined alga sample, wherein a predetermined solution quantity from an algae solution is drawn by means of a cuvette and a suction device over a moistened surface filter so as to place the algae contained in the solution on the surface filter and wherein the filter with the algae layer adhering thereto is then placed into the measuring device for determining the oxygen balance,
**characterized in that**
the algae solution substantially contains 0.25 to 5 million algae per ml of the solution, wherein the predetermined solution quantity of the sample is in the range between 3 ml to 8 ml, preferably 5 ml, so as to produce an algae monolayer on the filter surface.

2. Method according to claim 1,
**characterized in that**
a moistened or moist paper material or finely perforated film material is used as surface filter.

3. Method according to claim 1 or 2,
**characterized in that**
a syringe is used as suction device.

4. Method according to claim 3,
**characterized in that**
the syringe comprises a two-part cannula which receives an exchangeable filter leaf.

5. Method according to one of the preceding claims,
**characterized in that**
an airflow is passed over or though the filter to improve the adherence of the algae to the filter surface.

6. Method according to one of the preceding claims,
**characterized in that**
the algae solution and the possibly used airflow are temperature-controlled.

7. Method according to claim 6,
**characterized in that**
the temperature of the solution or of the airflow is in the range of 20°C to 28°C, preferably at 25°C.

## Revendications

1. Procédé pour déterminer la vitalité d'algues par dégagement d'oxygène lors de l'activité de photosynthèse par mesurage de leur bilan d'oxygène dans le processus de photosynthèse excité par voie artificielle d'un échantillon d'algues défini, dans lequel, à partir d'une solution d'algues, on tire une quantité prédéterminée de solution par-dessus un filtre surfacique humecté, au moyen d'une cuvette ainsi que d'un dispositif d'aspiration, de sorte que les algues situées dans la solution parviennent sur le filtre surfacique et ensuite on introduit dans le dispositif de mesurage le filtre comportant la couche d'algues qui y adhère pour déterminer le bilan d'oxygène,
**caractérisé en ce que**
la solution d'algues présente sensiblement 0,25 à 5 millions d'algues par ml de solution, la quantité prédéterminée de solution de l'échantillon étant dans la plage entre 3 ml à 8 ml, de préférence de 5 ml, afin de générer une monocouche d'algues sur la surface du filtre.

2. Procédé selon la revendication 1,
**caractérisé en ce que**
on utilise à titre de filtre surfacique un matériau de papier humecté ou humide ou un matériau en film à perforations fines.

3. Procédé selon la revendication 1 ou 2,
**caractérisé en ce que**
on utilise une seringue à titre de dispositif d'aspiration.

4. Procédé selon la revendication 3,
**caractérisé en ce que**
la seringue présente une canule en deux parties qui reçoit une feuille filtrante interchangeable.

5. Procédé selon l'une des revendications précédentes,
**caractérisé en ce que**
dans le but d'améliorer l'adhérence des algues sur la surface du filtre, on achemine un flux d'air par-dessus ou à travers le filtre.

6. Procédé selon l'une des revendications précédentes,
**caractérisé en ce que**
on met à température aussi bien la solution d'algues que le flux d'air utilisé le cas échéant.

7. Procédé selon la revendication 6,
**caractérisé en ce que**
la température de la solution ou du flux d'air est dans la plage de 20°C à 28°C, de préférence de 25°C.
